# EUROPEAN PATENT APPLICATION

(11) **EP 2 810 932 A1**
(43) Date of publication of application: **10.12.2014**
(21) Application number: 13170756.4
(22) Date of filing: 06.06.2013
(51) Int. Cl.: C07C 69/82, C08K 5/00, C08K 5/12

(54) **Improvements in or relating to plasticiser esters**

(71) Applicant: ExxonMobil Chemical Patents Inc., Baytown, TX 77520 (US)
(72) Inventor: Leroy, Bernard, 1341 Ceroux-Mousty (BE); Naert, Didier, 1200 Bruxelles (BE); Buess, Philippe Louis, 3090 Overijse (BE)
(74) Representative: Troch, Geneviève

(57) **Abstract**

Terephthalic acid di-alkyl esters in which the alkyl groups of the ester are obtained from branched C5 to C13 alcohols or mixtures of mixtures of C5 to C13 linear and branched alcohols in which less than 50 mole % of the alcohols are linear alcohols and the branched chain alkyl groups of such alcohols contain methyl groups and have an average of more than 7 carbon atoms and are free of ethyl groups. The di-esters are particularly useful as plasticizers and particularly plasticizers for polyvinyl chloride.

## Description

The present invention relates to improvements in or relating to esters, in particular esters useful for plasticising polyvinyl chloride.

### BACKGROUND OF THE INVENTION

Plasticizers are incorporated into a resin (usually a plastic or elastomer) to increase the flexibility, workability, or distensibility of the resin. The largest use of plasticizers is in the production of "plasticized" or flexible polyvinyl chloride (PVC) products. Typical uses of plasticized PVC include films, sheets, tubing, coated fabrics, wire and cable insulation and jacketing, toys, flooring materials such as vinyl sheet flooring or vinyl floor tiles, adhesives, sealants, inks, carpet backing, car underbody sealing and medical products such as blood bags and tubing.

Non limiting examples of other polymer systems that use plasticizers include polyvinyl butyral, acrylic polymers, poly(vinylidene chloride), nylon, polyolefins, polyurethanes, silicon modified polymers, polysulphides and certain fluoroplastics. Plasticizers also referred to as extenders in this context can also be used in rubber formulations.

Low molecular weight (LMW) phthalates, more simply known as low phthalates, include phthalic acid dialkyl esters having from 3 to 6 carbon atoms in their alkyl chains. The most common types of low phthalates include diethylhexylphthalate (DEHP), dibutylphthalate (DBP), di-isobutylphthalate (DIBP) and benzylbutylphthalate (BBP).

Esters of phthalic and terephthalic acid have been sold as plasticisers for many years and the esters of phthalic acid have found widespread use as plasticisers for polyvinyl chloride, Diisononyl phthalate (DINP) is widely used as a plasticiser and is commercially available from ExxonMobil as Jayflex™ DINP. Although phthalic acid esters have been more commonly used, terephthalic esters have also been proposed. For example, di-2-ethyl-hexylterephthalate (DEHTP), sometimes also referred to as dioctylterephthalate (DOTP) has been described as a suitable plasticizer since at least 1973 and is commercially available. US2005/0020718 suggests that alkyl terephthalates such as dimethylterephthalate, diethylterephthalate, di-n-propylterephthalate, di-n-butylterephthalate, di-isobutylterephthalate, monoglycol esters of terephthalic acid, diglycol esters of terephthalic acid, di-n-octyl terephthalate, diisooctyl terephthalate, di-2-ethylhexyl terephthalate, di-n-nonyl terephthalate, diisononyl terephthalate, di-n-decyl terephthalate, di-n-undecyl terephthalate, diisoundecyl terephthalate diisodecyl terephthalate, diisododecyl terephthalate, di-2-propyl heptyl terephthalate, di-n-octadecyl terephthalate, diisooctadecyl terephthalate, diisotridecyl terephthalate, di-n-eicosyl terephthalate, and or dicyclohexyl terephthalate may be used together with esters of cyclohexane carboxylic acids.

A number of patents teach the use of terephthalate esters having low carbon numbers in the alcohol moieties, e.g., C4-C5 alcohols, in compositions with PVC, such as US 7,361,779, which lowers the freezing point of butyl terephthalate by making n-butyl isobutyl terephthalate and US 2007/037926.

US 3,736,348, discloses plasticiser esters prepared from mixtures of ortho-phthalic acid, iso-phthalic acid and terephthalic acid.

WO 2008/140177 relates to compositions of terephthalic acid esters and their use as plasticisers. It relates to mixtures of two different diesters and an ester having mixed alkyl groups prepared by reacting terephthalic acid with a mixture of C3 to C12 alcohols. The ester mixtures contain from 3 to 77 mole % of each of the diesters and from 20 to 94 mol % of the mixed ester. The terephthalic esters are said to improve the properties of polymer resin moulded product including tensile strength, heating loss, elongation, age resistance and weatherability. In one embodiment three different alcohols may be used resulting in a more complex mixture. The alcohol mixtures used in the examples are different mixtures of the branched chain alcohols 2 ethylhexanol and isononyl alcohol. EP 2114854 describes the esterification of terephthalic acid with linear alcohols or 2-ethylhexanol.

US 2011/0281987 discloses C5-C7 alcohol diesters of terephthalic acid and their use as plasticisers in particular the di-C7 esters where the alcohol is Exxal™ 7.

Plasticisers are required to have a wide range of properties. Due to regulatory pressure, producers of flexible PVC articles are looking towards formulating away from low molecular weight ortho-phthalates to terephthalates. If ortho-phthalates are known to be low viscosity plasticizers, exhibiting low pour point or freezing point and exhibiting good gelation behaviour, tere-phthalates exhibit generally poorer performance as plasticizers.

Low molecular weight alcohol terephthalates di-esters (like for example the commercial plasticizer Eastman DBT, di-butylterephthalate) are known to be fast fusing plasticizers but such plasticizers freeze when stored at low temperature. Freezing of plasticizers close to room temperature require the installation of extra and costly handling equipment like for example heated tanks and transfer lines.

Higher alcohol terephthalates di-esters (C8 and above), like for example Eastman 168, are slow fusing, i.e. require higher processing temperatures than the corresponding orthophtalate diesters and offer lower levels of compatibility with PVC. The phthalate esters of high molecular weight linear alcohols exhibit good cold temperature properties but suffer also from a high freezing point.

We have found that certain mixtures of terephthalic acid di-esters can not only improve plasticizer storage life (reduce their freezing point and pour point) but provide also useful processing and performance advantages when used as plasticisers in polyvinyl chloride formulations. Terephthalic acid di-alkyl esters with short chain (C4 to C6) linear alkyl groups are solids at ambient temperature which is undesirable and we have found that if branched chain alcohols containing methyl branches and with an average number of carbon atoms above 7 are used for the preparation of the esters, then useful liquid products may be obtained.

### SUMMARY OF THE INVENTION

The present invention therefore provides terephthalic acid dialkyl esters in which the alkyl groups are branched alkyl groups or a mixture of branched and linear alkyl groups and in which the alkyl groups contain from 4 to 13 carbon atoms and the branched alkyl groups have an average of at least 7 carbon atoms and 50 % or less of the alkyl groups are linear and the branched alkyl groups contain methyl branches and contain less than 5% ethyl branches.

In a preferred embodiment the two terephthalic acid ester groups alkyl groups of differing carbon numbers. Preferably, the branched chain alkyl groups contain an average of more than 8 carbon atoms.

In a further embodiment the invention provides the use of such terephthalic acid dialkyl esters as plasticisers in polymer formulations and in particular in polyvinyl chloride (PVC) formulations.

In one aspect, the invention is therefore directed to compositions comprising at least one polymer and at least one plasticiser selected from the C4-C13 mixed alcohol terephthalic acid di-alkyl esters of this invention. The alkyl groups of the di-ester are either derived from branched alcohols or derived from a mixture of branched and linear alcohols containing 50% or less of linear alcohols, making a plasticiser containing 25% or less linear di-ester and the branched alcohols contain methyl branches and contain less than 5% of ethyl branches and preferably free from ethyl branches.

In embodiments, the invention is directed to plastisols comprising one or more of the terephthalic acid di-alkyl esters of this invention and PVC and/or another plasticizable polymer.

In a further embodiment the invention is directed to compounds comprising one or more of the terephthalic acid di-alkyl esters of this invention and PVC and/or another plasticizable polymer which can be prepared by mixing the terephthalate diesters with suspension PVC.

In a further embodiment the invention is directed to PVC plastisols comprising one or more of the terephthalic acid di-alkyl esters of this invention to lower the PVC plastisol viscosities and improve their gelation.

In a further embodiment the invention is directed to flexible PVC compositions where one or more of the terephthalic acid di-alkyl esters of this invention are used as fast fusing plasticizers.

In a further embodiment the invention is directed to flexible PVC compositions where one or more of the terephthalic acid di-alkyl esters of this invention are used as a fast fusing plasticizer in combination with a slower fusing plasticizer (i.e. a plasticizer with higher gelation temperature) which may be a phthalate or an non-phthalate plasticizer like diisononyl cyclohexanediacid ester or an epoxidized oil plasticizer, or dibasic acid esters like adipates, succinates or azelates or any other vegetable oil derived plasticizer or a polymeric plasticizer, di-benzoates or mono benzoates esters or a trimellitate.

In a further embodiment the invention is directed to sealants, caulks and mastics comprising the terephthalic acid di-alkyl esters of this invention.

### DETAILED DESCRIPTION OF THE INVENTION

The present invention provides terephthalate diesters suitable for use as plasticizers for a wide variety of polymer compositions. In general the polymer will be a plasticizable polymer (or resin) and the terephthalate ester additive according to this invention will act as a plasticizer and thus will be present in normal plasticizing amounts. However, as will be made clear below, the terephthalate ester additive according to the invention will act, in embodiments, as something other than a plasticizer or in other cases as both a plasticizer and for some other function. Accordingly, it will be understood that the present inventors do not wish to be bound by theory as to whether such "additives" act as a plasticizer or not. It will be understood also that, unless otherwise specified, the additive of the invention may also be present as a co-plasticizer, i.e., at least one other plasticizing ester is at least partially for plasticizing purposes. This at least one other plasticizing ester may be a different terephthalate ester or it may be a non-terephthalate ester, or a combination thereof.

The polymer employed in the plasticized formulations of this invention may be selected from PVC, butyl rubbers, acrylics, methacrylates, fluoroplastics, urethanes, polyurethanes, polysulphides, silicones, block copolymers, silicone modified polymers, and mixtures thereof. The invention is particularly directed to formulations in which the polymer is PVC.

The terephthalate esters of the present invention are prepared by esterifying terephthalic acid with branched alcohols or a mixture of linear and branched alcohols or by trans-esterification of dimethyl terephthalate with branched alcohols or a mixture of branched and linear alcohols. The alcohols contain 50 mole % or less linear alcohols; the branched alcohols contain methyl branches and are free of ethyl branches. The esters may be prepared by esterification or trans-esterification with an alcohol mixture containing branched C4 to C 13 alcohols in which from 10 to 49 mole % (based on the alcohol mixture) are linear C4 to C 13 alcohols wherein the branched alcohols contain methyl branches and no ethyl branches and contain an average of more than seven carbon atoms. The alcohols preferably contain alkyl groups of different numbers of carbon atoms. The alcohols will react with the terephthalic acid or dimethyl terephthalate somewhat at random and so the esterification reaction will produce some di-esters containing the same alkyl groups and some di-esters containing mixtures of alkyl groups and some may have all linear alkyl groups and some may have all branched alkyl groups. It is preferred that the amount of di-esters containing linear alkyl groups be lower than 25%.

Preferred branched alcohols that can be included in the alcohol mixtures includes that are reacted with terephthalic acid include 2-methyl butanol, 3-methyl butanol, 2,2-dimethyl propanol, 2-methyl pentanol, 3-methyl pentanol, 4-methyl pentanol, 2,3-dimethyl butanol, 2-ethyl butanol, 2-methyl hexanol, 3-methyl hexanol, 4-methyl hexanol, 5-methyl hexanol, 2,3-dimethyl pentanol, 2,4-dimethyl pentanol, 3,4-dimethyl pentanol, branched C8 alcohols, dimethyl hexanol, branched C9 alcohols, branched C10 alcohols, branched C11 alcohols and branched C13 alcohols all containing methyl branches and being free of ethyl branches. The C8, C9, C10, C11 and C13 alcohols may be used on their own. The lower carbon number alcohols with branched alkyl groups are used in mixtures with higher carbon number alcohols to provide branched chain alcohols with an average carbon number above seven. The alcohols may be mixtures of isomers such as those sold by ExxonMobil under the Exxal™ brand name.

When used, linear alcohols are preferably selected from the group consisting of n-butanol, n-pentanol, n-hexanol, n-heptanol, n-octanol, n-nonanol, n-decanol , n-undecanol, n-dodecanol and n-tridecanol. This invention provides liquid esters with linear alkyl groups derived from such linear alcohols.

Proton-NMR(¹H-NMR) at room temperature can be used to determine the average composition of a phthalate plasticizer providing useful information like the average carbon number and the average number of branches per molecule. FT-NMR-instruments (300 MHz or higher) are used in the characterization of plasticizers. Typically a 10 % solution of the material in CDCl₃ is used and the ¹H-NMR spectrum is obtained at room-temperature, making sure a small pulse angle and an adequate delay between pulses are used.

The table below shows the carbon number distribution (CND) and the average number of branches per molecule of Exxal™ 7, Exxal™ 8, Exxal™ 9 and Exxal™ 10. For carbon number distribution (CND) determination of Exxal™ 8 (and Exxal™13), a Hewlett Packard 5890 series II Chromatograph can be used. For Exxal™ 7, Exxal™ 9 and Exxal™ 10 (and Exxal™ 11) a Hewlett Packard 6890 Chromatograph can be used.

| Carbon number distribution (CND): Typical values measured by Gas Chromatography | | | | | | | | Ave. Number of Branch/molecule (¹NMR) |
|---|---|---|---|---|---|---|---|---|
| Percentage | C6 | C7 | C8 | C9 | C10 | C11 | CND | |
| Exxal7 | 4 | 83 | 13 | | | | 7.1 | 1.2 |
| Exxal8 | | 2 | 93 | 5 | | | 8.0 | 1.6 |
| Exxal9 | | | 3 | 77 | 20 | | 9.2 | 2.1 |
| Exxal10 | | | | 3 | 90 | 7 | 10.0 | 2.0 |

The alcohols used in preparation of the terephthalate esters of the present invention contain essentially only methyl branches and are essentially free of ethyl groups, having less than 5%, preferably less than 3% ethyl groups.

The most important uses of compositions employing the terephthalic acid di-alkyl esters of the present invention are PVC plastisols, which can be molded, spread, sprayed, coated, dipped or processed into a variety of end use applications including vinyl sheet flooring, cushioned vinyl, toys, wall coverings, synthetic leather and other fabrics and as dry blends or flexible PVC compounds in compounding, calendaring, extrusion, powder coating applications.

The composition according to the invention maybe prepared in the form of a plastisol, that is, a dispersion of finely divided resin in the plasticizers of the invention, and then processed in a manner appropriate for the end use.

Plastisols are generally dispersions of organic polymers in plasticizers which gel and fuse on heating to relatively high temperatures of about 170°C to 190°C and cure on cooling to form the flexible PVC product. Plastisols typically comprise finely powdered polyvinyl chloride. Additionally they can contain copolymers of vinyl chloride and, more recently, methacrylate copolymers or styrene copolymers. These finely powdered polymers are dispersed in a liquid plasticizer and form the paste-like plastisol. The plastisols are used for various applications. They may be used, for example, as sealing compounds, for impregnating and coating substrates of textile materials, and as adhesives. In the automotive industry, plastisols according to the invention may be used for interior applications like coated fabrics or dashboards, for under body protection, for sealing seams, for lining hoods, as vibration-damping materials or as adhesives. Other products prepared from plastisols include toys, vinyl flooring, vinyl gloves, carpet backing, floor mats, non woven or polyester based coated fabrics for wall coverings or tarpaulins, tool handles, end caps, and the like.

Plastisols are useful in the production of flooring, tents, tarpaulins, coated fabrics such as automobile upholstery, in car underbody coatings, in mouldings and other consumer products. Plastisols are also used in medical uses such as blood bags and tubing, footwear, fabric coating, toys, flooring products and wallpaper. Plastisols typically contain 30 to 200 parts by weight, more typically 35 to 150 parts by weight, more typically 40 to 120 parts by weight, more typically 50 to 90 parts by weight of plasticiser per 100 parts of polyvinyl chloride.

Plastisols are usually made from polyvinyl chloride that has been produced by emulsion polymerisation or micro suspension polymerisation. The plastisol may be produced by the manufacturer of the polyvinyl chloride or a compounder and shipped to the user in fluid form. Alternatively the plastisol may be produced by the user. In either instance, although particularly when the plastisol is produced by the manufacture of the polyvinyl chloride or a compounder, it is important that the plastisol viscosity be stable over time.

The present invention therefore provides a plastisol composition containing from 30 to 200 parts by weight, preferably 35 to 150 parts by weight, more preferably 40 to 120 parts by weight, more preferably 50 to 90 parts by weight of plasticizer per 100 parts of polyvinyl chloride, wherein the plasticizer comprises one or more terephthalic acid di-alkyl esters of this invention.

In a further embodiment, the present invention provides a process for the production of flexible polyvinyl chloride comprising forming a layer from a plastisol containing from 30 to 200 parts by weight preferably 35 to 150 parts by weight, more preferably 40 to 120 parts by weight, more preferably 50 to 90 parts by weight of a plasticiser composition containing one or more terephthalic acid di-alkyl esters of this invention per 100 parts by weight of polyvinyl chloride and subsequently fusing the layer by the application of heat.

Depending on the particular application envisaged, the plastisols may contain additives in addition to the fine-particle polymer powders and the liquid plasticizers. These other additives include, for example, fillers, coupling agents, lubricants, stabilizers, flow aids, water-absorbing substances, pigments or blowing agents.

One of the advantages of the present invention is that, the use of the terephthalic acid di-alkyl esters of this invention meets the criteria of low volatility, low freezing point and melting point, faster gelation and fusion at a lower temperature and/or good solvating strength , good plastisol viscosity properties that do not change much upon storage, and provides a good flexible PVC product with good low temperature properties. The terephthalic acid dialkyl esters of this invention exhibit also good plasticizer efficiency i.e. the same hardness level is achieved with lower plasticizer concentration, compared to corresponding ortho-phthalic acid di-alkyl esters.

One advantageous use of the terephthalic acid di-alkyl esters of this invention is as a modifier for mastics, caulks, and sealants. Mastics, caulks, and sealants are used to seal off or to provide a flexible joint between different materials to exclude dust, dirt, moisture, chemicals, and the like, to reduce noise and vibration, to insulate or to serve as space fillers. While often based on phthalates or alkylsulfonic esters of phenol (e.g., Mesamoll™ from Bayer), an increasing amount of sealant and mastics is produced using polyurethane, acrylics, block copolymers, silicones, or polysulphides. Typically any and all of these base polymer systems contain various amounts of plasticizer depending on the specific composition, processing, and end use, but generally the amount of plasticizer is in the range of 1 to 30 % by weight of the total system weight. The terephthalic acid di-alkyl esters of this invention are useful plasticizers for these mastics and sealants. With acrylic mastics, caulks, and sealants, in addition to providing a role of making the sealant flexible, the di-esters of this invention are useful coalescing additives.

The terephthalic acid di-alkyl esters of this invention have also been found to be useful as viscosity modifiers, wetting agents for fillers and substrates, particularly as a wetting agent for applying mastics, sealants, and other coatings. More generally, compositions containing fillers, pigments, lubricants, stabilizers, viscosity depressants, blowing agents, adhesion promoters, and flame retardants, and/or compositions which are to be applied (or can be applied) by knife, gun, manual or metered dispenser can benefit if the di-esters of this invention are present in the composition.

The terephthalic acid di-alkyl esters of this invention may be used as plasticisers across the range of plasticised polyvinyl chloride materials and they are applicable to the production of semi-rigid polyvinyl chloride compositions which typically contain from 10 to 50 parts, preferably 15 to 35 parts, more preferably 20 to 30 parts of plasticiser per 100 parts of polyvinyl chloride. The di-esters are also useful in flexible polyvinyl chloride compositions which typically contain from 40 to 100 parts preferably 45 to 80 parts, more preferably from 48 to 52 parts per 100 parts of polyvinyl chloride and also to the highly flexible compositions which typically contain from 70 to 110 parts, preferably 80 to 100 parts, more preferably 90 to 100 parts of plasticiser per 100 parts of polyvinyl chloride. The parts being by weight.

The semi-rigid compositions are typically used for the production of pipes, some wire and cable coatings, floor tiles, window shades, films, blood bags and medical tubing. Flexible compositions are typically used for the production of sheeting, upholstery, medical tubing, garden hoses, pool liners, water beds and the like. Very flexible compositions are used in the production of coated cloth, toys, shoe soles and the like. The terephthalic acid dialkyl esters of this invention are particularly useful in the production of medical articles such as blood bags and medical tubing and in toys and materials used for food contact such as bottle caps and films where di-2-ethyhexyl phthalate has traditionally been used and there are some concerns about its toxicity have been expressed.

In a further embodiment, the invention provides roofing, tarpaulins, tents, films, sheeting, floor covering, shoes and automobile interiors obtained from a plasticised polyvinyl chloride composition containing from 20 to 100 parts by weight preferably 30 to 90 parts by weight, more preferably 40 to 80 parts by weight, more preferably 50 to 70 parts by weight of a plasticiser composition containing one or more terephthalic acid di-alkyl esters of this invention per 100 parts of polyvinyl chloride.

The use of the terephthalic acid di-alkyl esters of this invention as plasticisers for polyvinyl chloride compositions also provides improved cold flex properties. Cold flex leads to an improved service temperature range and is particularly useful in the production of articles used in a wide range of temperatures. Throughout this application the cold flex properties are measured using the Clash and Berg test (ASTM D 1043-84) and the ASTM D 746 brittleness test. The improved cold flex is particularly useful when the plasticised polyvinyl chloride composition is to be used in articles which are used over a wide temperature range. In particular this is useful in applications such as roofing, tarpaulins and tents, protective films including food wrap films, wire and cable, coated fabrics, shoes and medical applications such as blood bags and tubing.

The workability of these plastisols can still leave something to be desired and for applications, such as flexible floor coverings it is usual to include additives which will depress the viscosity of the plastisol to an even lower level than that achieved by using only conventional plasticisers. Lower levels of viscosity are commonly achieved by the inclusion of viscosity depressants which are often hydrocarbon fluids such as Exxsol™ D100 or D140 sold by ExxonMobil. However these materials are increasingly regarded as unacceptable, particularly in applications such as floor coverings, because the finished articles release noticeable amounts of volatile materials when they are stored or are in use at room temperature.

The problem of increased emissions is exacerbated in applications such as floor coverings which are laminates of several layers of polyvinyl chloride because it is desirable to minimise the amount of plasticiser present in the top coating of the floor covering to improve the wearing properties and stain resistance. For ease of processing it is then necessary to use a high level of viscosity depressant. It would therefore be highly desirable to be able to reduce the viscosity of the plastisol during processing without encountering the problem of increasing the emissions of volatile organic compounds from the finished articles.

Viscosity control is important in the conversion of these plastisols into useful products. For example in the preparation of vinyl floor coverings, the plastisol is spread on a surface moving at around 15 to 25 meters per minute in several layers so that the floor covering is literally built up. Typically these layers include a foam core, a decorative layer and a clear protective wear layer. The multilayer products are first gelled by contact with a heated roll and then passed into an oven where they are fused (gelled) at a temperature of from 180°C to 200°C. Often the gelling is performed after the spreading of each individual layer, starting with the base or encapsulation layer. After the gelling, the next layer can be spread. When all layers have been spread, the product is then passed into an oven to obtain full fusion of all layers together and adequate expansion of the foamed layers.

To fulfill the plastisol spread coating requirements in terms of production speed (adequate viscosity and adequate gelation) a wide range of viscosity regulators, usually viscosity depressants, can be used.

Traditional viscosity depressants used today include hydrocarbon fluids, such as Exxsol™ D100 or Exxsol™ D140 sold by ExxonMobil, or linear alkyl benzene or 2,2,4-trimethylpentanediol diisobutyrate, sold as Texanol by Eastman Chemical or specialty esters based on oleates and laurates. WO97/35060 discloses a plastisol composition comprising a chlorine-containing resin, a primary plasticiser and a C 11-C14 straight chain or branched chain alkyl benzoate.

Although the above products perform well, they have limited compatibility and are therefore only useful at low plasticizer concentrations. They also have high volatility, and their effect on gelation temperature, their little or no plasticising effect, their slow fusion with the resin, their cost performance ratio and their contribution to the Volatile Organic Carbon (VOC) emissions from finished products has led to a search for improved products.

We have also found that the use of the terephthalic acid di-alkyl esters of this invention as plasticisers for polyvinyl chloride also results in improved processability of the polyvinyl chloride compositions. This improved processability is particularly useful in the transformation of the plasticised polyvinyl chloride composition. Transformations include, for example, pelletising, extrusion, injection moulding and calendering. Calendering is used in applications such as the production of roofing, protective films including stationery. Extrusion is used in the production of films, pipes, guttering and wire and cable coatings. Injection moulding is used in the production of shoes, toys and the like.

In a further embodiment, the invention provides extruded articles obtained from a plasticised polyvinyl chloride composition containing from 20 to 100, preferably from 30 to 90, more preferably from 40 to 80, more preferably from 50 to 70 parts by weight of a plasticiser composition containing one or more terephthalic acid di-alkyl esters of this invention per 100 parts of polyvinyl chloride.

In a further embodiment the invention provides pellets comprising polyvinyl chloride and from 10 to 100, preferably from 30 to 90, more preferably from 40 to 80, more preferably from 50 to 70 parts by weight of a plasticiser composition containing one or more terephthalic acid di-alkyl esters of this invention per 100 parts of polyvinyl chloride.

In a further embodiment the invention provides injection moulded articles obtained from a plasticised polyvinyl chloride composition containing from 20 to 100, preferably from 30 to 90, more preferably from 40 to 80, more preferably from 50 to 70 parts by weight of this invention terephthalic acid di-alkyl esters of this invention per 100 parts of polyvinyl chloride.

The use of esters of terephthalic acid as plasticisers for polyvinyl chloride can result in an increase in the temperature required for gelation compared to compositions based on comparable phthalates. Accordingly in a further embodiment of the invention fast fusing plasticisers can be added to the composition containing the di-ester of terephthalic acid according to this invention.

The esters of this invention can be used such as for the manufacture of wall coverings, flooring, toys, conveyor belts, synthetic leather. Typical formulations could be, in parts by weight

| | |
|---|---|
| PVC resin | 100 |
| Fast fusing plasticizer | 5-25 |
| Terephthalic acid di-ester of the invention | 30-50 |
| Filler | 0-50 |
| Stabilizer | 1-4 |
| Other | 0-10 |

Alternatively, typical formulations for use in the production of automotive underbody sealants which typically have high plasticiser and high filler contents in parts by weight, are

| | |
|---|---|
| PVC or PVC copolymer (or combinations of the two) | 100 |
| Fast fusing plasticiser | 20-35 |
| 1,4 terephthalic acid di-ester of the invention | 60-90 |
| Filler such as calcium carbonate | 80-150 |
| Stabilizer and other additives | 0-10 |

As a further embodiment formulations for the production of calendered floor tiles in parts by weight are

| | |
|---|---|
| PVC or PVC copolymer (or combinations of the two) | 100 |
| Fast fusing Plasticiser | 10-30 |
| 1,4 terephthalic acid di-ester of the invention2 | 0-30 |
| Epoxidized soybean oil | 0-6 |
| Filler e.g. CaCO₃ | 500-800 |
| Pigments, stabilizers, other additives | 0-10 or as needed. |

The plasticizers of the present invention can also be used for thick extruded materials such as those used for the manufacture of thick chair mats, water stops, and extruded profiles. For applications where the extruded material may be thicker than 3-4 mm, if the plasticised PVC is not thoroughly mixed in the extruder, the surface is blemished, sometimes dull, sometimes containing mold marks, waves, or streaks. Adding a little fast fusing plasticiser to the plasticiser blend can correct this problem. Examples of suitable formulations for extrusions in parts per hundred are

| | |
|---|---|
| PVC | 100 |
| Fast fusing plasticiser | 5-15 |
| Terephthalic acid di-ester of the invention | 10-30 |
| Filler | 0 - 25 |

together with pigments, lubricants, stabilizers, other additives, as needed.

Examples of non-phthalate fast fusing plasticisers which can be used in any of the above mentioned applications include diethylene glycol dibenzoate, dipropylene glycol dibenzoate, isononyl benzoate, isodecyl benzoate, 2-ethyl hexyl benzoate, alkyl sulfonic esters of phenol available from Bayer as Mesamoll, citrates such as tributylacetyl citrate, tri-2-ethylhexyl phosphate, trioctyl phosphate such as 2-ethylhexyl-isodecyl phosphate, di-2-ethylhexyl phenyl phosphate, triphenyl phosphate, tricresyl phosphate.

High chlorine content chlorinated paraffins are also known to reduce fluxing temperatures. Materials containing 60 to 70 wt % chlorine can have fusion temperatures between 75 and 84°C, while the lower chlorine materials, containing 40 to 54 wt % chlorine, can have fluxing temperatures around 120-135°C.

Low molecular weight diesters such as dibutyl adipate also reduce fusion/flux temperatures although it is preferable according to this invention to use no more than 10 parts per hundred of those materials because larger amounts could create an undesirable rise in volatility.

In the embodiments of the present invention reference to systems containing the terephthalic di-alkyl esters of this invention are to systems in which the terephthalic acid dialkyl ester is the sole plasticiser and also to systems in which it is present in admixture with other plasticisers, for example fast fusing plasticizer.

The polyvinyl chloride and the terephthalic acid di-alkyl ester of this invention may be mixed by the conventional formulating techniques currently used in the production of plasticised polyvinyl chloride formulations. The formulator will attempt to provide a versatile composition having a good balance of properties at reasonable cost. The formulator will be concerned to optimise the balance between end-product properties such as flexibility, low temperature performance, flame resistance, high temperature resistance, volatility, stain resistance, electrical properties and processability and the processing properties such as plastisol viscosity, fusion, dry blending, emissions and printability.

In another aspect of the present invention the terephthalic acid di-alkyl esters of this invention are used together with other plasticisers. For example, they may be used with plasticisers like dibasic acid esters such as adipates, succinates, azelates, sebacates, orthophthalate esters, trimellitate esters, mono benzoate or di-benzoate esters, various polymeric plasticisers or any vegetable oil derived plasticizers, whether, from soy bean oil , castor oil some of which have been described previously. When used in plasticiser blends the relative proportions of the plasticisers that are used will depend upon the desired properties. Preferably, however the terepthalic acid di-alkyl esters of the invention represent at least 5 wt %, more preferably at least 10 wt %, more preferably at least 15 wt %, more preferably at least 20 wt %, more preferably at least 25 wt %, more preferably at least 30 wt %, more preferably at least 35 wt %, more preferably at least 40 wt %, more preferably at least 45 wt %, more preferably at least 50 wt %, more preferably at least 55 wt %, more preferably at least 60 wt %, more preferably at least 65 wt %, more preferably at least 70 wt %, more preferably at least 75 wt %, more preferably at least 80 wt %, more preferably at least 85 wt %, more preferably at least 90 wt %, of the total weight of plasticiser present in the plasticizer composition.

The formulations containing the polyvinyl chloride and the plasticiser may contain other additives. The majority of formulations will contain a stabiliser which counters the effects of ageing; heat stabilisers also reduce the dehydrodehalogenation of the polyvinyl chloride at the temperatures at which the formulation is processed. Stabilisers, such as benzotriazole and benzophenone, also reduce the degradation by sunlight, ozone and biological agents. The improved ultra-violet stability obtained by the use of the esters of the cyclohexane polycarboxylic acids according to the present invention may enable smaller amounts of stabilisers to be used. Typically, the formulations contain from 0.5 to 10 parts, normally from 1.5 to 3 parts, by weight of stabiliser per 100 parts of the polyvinyl chloride.

Stabilisers that provide stability during heat processing are typically metal compounds. For wire and cable applications, organotin compounds, barium, cadmium and zinc salts or calcium/zinc stabilisers may be used. Organic phosphates and polyols may also be used. Lead stabilisers may also be used in wire and cable applications. Calcium/zinc stabiliser systems are also used in foil and sheeting, wall coverings, medical applications, tubes and footwear, food packaging film and fabric coating. Barium/zinc stabiliser systems are used in foil and sheeting, flooring, wall covering, tubes and footwear and fabric coating. Tin stabilisers are used in flooring and wall covering. Zinc compounds are frequently used as a stabiliser and as kickers (agents which control the composition of blowing agents) in formulations used to produce foams in, for example, flooring, wall covering and fabric coating.

Other ingredients which may be added to the polyvinyl chloride formulations include fillers such as calcium carbonate, titanium dioxide or silica. When used, the filler may be present in an amount up to 250 parts, preferably up to 100 parts per 100 parts of polyvinyl chloride. Lubricants, pigments and processing acids may be included. Other ingredients will be chosen according to the use to which the formulation is to be put. For example, the formulation may contain flame retardants, blowing agents and kickers, biostabilisers, antistatic agents, viscosity regulators such as thickeners and thinners, antifogging agents which are particular useful is packaging films and antioxidants, such as bisphenol A.

Fillers are incorporated in the formulations to reduce cost, increase the output of dry blending, increase electrical resistance, increase resistance to ultra-violet light, increase hardness, produce improved heat transmission, increase the resistance to heat deformation. Fillers can also impart anti-blocking or anti-slip performance. Examples of suitable fillers include calcium carbonate, clays such as alumino-silicates, silica, dolomite and bauxite.

The particular particle size distribution and average surface area of the filler will be chosen according to the properties it is desired to impart.

Lubricants and processing aids may be included to reduce the adhesion between polyvinyl chloride and hot machinery surfaces during processing. The lubricants also affect the frictional properties between resin particles during processing. Examples of lubricants include stearic acid and metal stearates which can also act as stabilisers. Other lubricants that may be used include petroleum waxes, silicon oil, mineral oil, synthetic oils and polyethylene waxes.

The formulations may also contain flame retardants to increase ignition time, reduce flame spreading and rate of burning. The flame retardants should have a high decomposition temperature, low volatility, a minimum effect on thermal and mechanical properties and good resistance to light and ultra-violet radiation. Examples of flame retardants that may be used include halogen containing compounds and phosphorous containing organic compounds such as triaryl, trialkyl or alkyl diaryl phosphate esters. Other materials that may be used include chloroparaffins, aluminum trihydrate Al(OH)₃ or antimony oxides Sb₂O₃.

Where the formulations are used to produce foams such as in flooring materials, they can contain a blowing agent which decomposes with the evolution of gas bubbles during processing of the plastisol. Examples of suitable blowing agents include azodicarbonamide which releases nitrogen when heated to a temperature in the range 200°C to 250°C. The system may also contain kickers which control and lower the decomposition temperature of the blowing agent. For example lead compounds such as dibasic lead phthalate, zinc oxide or barium/cadmium compounds may be used to reduce the activation temperature of azodicarbonamide to a temperature in the range 150°C to 215°C. These metal compounds can also act as stabilisers.

The invention is illustrated but in no way limited by the following Examples.

### Example 1 Direct esterification of terephthalic acid.

Into a five-necked, 2000 ml round bottom flask equipped with a mechanical stirrer, nitrogen inductor, thermometer, Dean-Stark trap and chilled water cooled condenser were added 2 moles of terephthalic acid (TPA) and 5 moles of Isoheptyl alcohol (Exxal™ 7) available from ExxonMobil. Tetra isooctyl titanate (TIOT, 1%) was used as catalyst. The Dean-Stark trap was filled with alcohol. The reaction mixture was heated to 210-220°C under a nitrogen sweep during a few hours. The water evolved during the esterification reaction was collected in the Dean-Stark trap and was drained frequently. After sampling for complete conversion, the reactor was cooled to 90°C and a sodium carbonate solution was added to hydrolyse and/or neutralize catalyst residues and neutralize any residual monoester. The excess alcohol and residual water were removed by steam and nitrogen stripping. The diheptyl terephthalate obtained could be used in mixtures with high carbon number esters according to the invention.

### Example 2 Transesterification from di-methylterephthalate (DMT).

Into a five-necked, 2000 ml round bottom flask equipped with a mechanical stirrer, nitrogen inductor, thermometer, Dean-Stark trap and chilled water cooled condenser were added 2 moles of DMT and 5 moles of isoheptyl alcohol (Exxal™ 7). Tetra n-butyl titanate (TnBT, 0.5%) was used as catalyst. The Dean-Stark trap was left empty. The reaction mixture was heated to between 130-180°C under a nitrogen sweep during 1 hour. The MeOH evolved during the transesterification reaction was collected in the Dean-Stark with the Exxal™ 7. The amount of MeOH in Exxal™ 7 was monitored by Gas Chromatography. After sampling, the reaction was stopped when the residual level of methylheptyl ester was below 0.3%.

The reactor was cooled to 90°C and a sodium carbonate solution was added to hydrolyses and/or neutralizes catalyst residues and neutralize the reaction mixture. The excess alcohol and residual water were removed by steam and nitrogen stripping.

### Examples 3 to 12 - Terephthalates

The products in the following Examples were prepared by a standard esterification process using an excess of the alcohol or alcohol mixture per mole of terephthalic acid and a titanium catalyst as is described in Example 1.

The melting and freezing (crystalisation) point of the esters so produced were obtained by DSC and the pour point was measured according to ASTM D5950 with the following results as shown in Table 1.

**Table 1 - Mixed Terephthalic Diester Properties**

| Examples | Alcohol | Di-ester Freezing Point (°C) | Di-ester Melting Point (°C) | Di-ester Pour Point (°C) |
|---|---|---|---|---|
| 3 | n-C8/Iso C8 + (50/50) | -32 | 23 | -3 |
| 4 | Iso C7/Iso C9* (50/50) | Not determined | Not determined | -52 |
| 5 | n-C8/Iso C10 ++ (50/50) | Not determined | 17 | -20 |
| 6 | Iso C9 * | Tg=-79.5 | No transition | -49 |
| 7 | Iso C10 ++ | Tg=-77.7 | No transition | -46 |
| 8 ⁽¹⁾ | n-C8/Iso C8 + (90/10) | 28 | 38 | 37 |
| 9 ⁽¹⁾ | n-C8/Iso C8 + (70/30) | 24 | 37 | 28 |
| 10 ⁽¹⁾ | n-C6 | 21 | 34 | 30 |
| 11 ⁽¹⁾ | n-C8 | Not determined | Not determined | 32 |
| 12 ⁽¹⁾ | n-C4 | -10 | 22 | 4 |

| | | | | |
|---|---|---|---|---|
| ⁽¹⁾ Comparative + Commercial Exxal™ 8 alcohol * Commercial Exxal™ 9 alcohol ++ Commercial Exxal™ 10 alcohol The Iso C7 alcohol was the commercial alcohol Exxal™ 7 | | | | |

The n-C4, n-C6, n-C8 and the mixtures of 70/30 and 90/10, n-C8/Iso C8 alcohols were employed for comparative purposes. The results show that with the higher proportion of normal alcohols, the freezing point, melting point and pour point are unacceptably high and that for the terephthalates di-alkyl esters where the ratio of linear to branched alcohol is below 50%, the resulting pour points are well below 0°C.

### Example 13

The solution temperature of the plasticisers of the invention and their gelation curve was compared with those of the corresponding ortho phthalates.

The formulations tested (plastisols) contained 100 parts PVC (Solvin 382G), 60 parts of ester plasticiser, and 1 part of a conventional stabiliser

The solution temperature of plasticizers is defined as the temperature at which a set amount of PVC gets dissolved in a set amount of plasticizer. The solution temperature is not only influenced by the plasticizer type but also by the PVC resin type and in particular the K-Value (DIN 53408 Testing of Plastics; Determination of Solubility Temperature of Polyvinyl Chloride (PVC) in Plasticizers (1967.06.01)).

The gelation temperature of the plastisols was determined by an Anton Paar Physica Rheometer MCR 301. The instrument was used in oscillation mode, frequency 1hz, amplitude 0.01% and the heating rate is 10°C/min. The gelation temperature is reported when G' (Elastic modulus) is equal to 10⁴ Pa.

The results were as follows.

**Table 2 - Plasticizer Gelation and Solution Temperatures**

| Terephthalates | Solution T in °C | Gelation T in °C | Ortho-phthalates | Solution T in °C | Gelation T in °C |
|---|---|---|---|---|---|
| DOTP | 130 | 112 | DOOP | 120 | 90 |
| DINTP (Ex. 6) | 147 | 137 | DINOP | 126 | 99 |
| DIDTP (Ex. 7) | 149 | | DIDOP | 132 | 111 |
| DIHNTP(*) (Ex. 4) | 143 | 125 | | | |

DIN means diisononyl and DID means diisodecyl, TP means terephthalate and OP means ortho phthalate. For DO (dioctyl), the ester is based on 2-ethyl hexanol.

(*) ester based on a 50/50 mixture of the C7 and C9 commercial branched alcohols Exxal™ 7 and Exxal™ 9.

## Claims

1. Terephthalic acid di-alkyl esters in which the alkyl groups are branched alkyl groups or mixed branched and linear alkyl groups containing from 4 to 13 carbon atoms wherein the branched chain alkyl groups contain an average of more than 7 carbon atoms and 50 % or less of the alkyl groups are linear and the branched alkyl groups contain methyl groups and less than 5% ethyl groups.

2. Terephthalic acid di-alkyl esters according to Claim 1 in which the amount of di-alkyl esters containing linear alkyl groups is lower than 25%.

3. Terephthalic acid di-alkyl esters according to Claim 1 or Claim 2 in which the alkyl groups contain differing numbers of carbon atoms.

4. Terephthalic acid di-alkyl esters according to any preceding claim, wherein the branched chain alkyl groups contain an average of more than 8 carbon atoms.

5. A composition comprising at least one polymer and at least one plasticiser comprising a di-alkyl ester according to any of the preceding claims.

6. A composition according to Claim 3 or Claim 4 comprising a plastisol comprising one or more terephthalic acid di-alkyl esters according to Claim 2 and polyvinyl chloride (PVC).

7. A flexible PVC composition where one or more of the terephthalic acid di-alkyl esters according to any of Claims 1 to 4 are used as a fast fusing plasticizer.

8. A PVC composition according to Claim 7 further comprising a slower fusing plasticizer.

9. A PVC composition according to Claim 8, wherein the slower fusing plasticizer is a phthalate or non-phthalate plasticizer, preferably a diisononyl cyclohexanediacid ester or an epoxidized oil plasticizer, or dibasic acid esters including adipates, succinates or azelates or any other vegetable oil derived plasticizer or a polymeric plasticizer, di-benzoates or mono benzoates esters or a trimellitate.

10. A flexible PVC composition where one or more of the terephthalic acid di-alkyl esters according to any of Claims 1 to 4 is blended with a fast fusing plasticizer.

11. A PVC composition according to claim 10, wherein the fast fusing plasticizer is selected from diethylene glycol dibenzoate, dipropylene glycol dibenzoate, isononyl benzoate, isodecyl benzoate, 2-ethyl hexyl benzoate, alkyl sulfonic esters of phenol, citrates such as tributylacetyl citrate, tri-2-ethylhexyl phosphate, trioctyl phosphate such as 2-ethylhexyl-isodecyl phosphate, di-2-ethylhexyl phenyl phosphate, triphenyl phosphate and tricresyl phosphate.

12. A composition according to any of Claims 5 to 11 in which the polymer is PVC having a degree of polymerization (DP) which is between 650 and 1600.

13. A composition according to any of Claims 5 to 12 in which the PVC is a suspension polymerised polyvinyl chloride of agglomerated particles of size in the range 80 to 200 microns.

14. A composition according to any of Claims 5 to 12 in which the PVC is emulsion polymerised.

15. A composition according to any of Claims 5 to 13 in which the PVC is a suspension or emulsion polymerised polyvinyl chloride having a K value in the range 62 to 70.

16. A composition comprising a plastisol containing 40 to 200 parts by weight, a terephthalic acid di-alkyl ester according to any of Claims 1 to 4 as a plasticiser per 100 parts of PVC.

17. A semi-rigid PVC composition containing from 20 to 80 parts by weight of a terephthalic acid di-alkyl ester according to any of Claims 1 to 4 as plasticiser per 100 parts of PVC.

18. A flexible PVC composition containing from 40 to 100 parts by weight of a terephthalic acid di-alkyl ester according to any of Claims 1 to 4 per 100 parts of PVC.

19. Roofing, tarpaulins, tents, films, sheeting, floor covering, shoes and automobile interiors obtained from a plasticised PVC composition containing from 20 to 100 parts by weight of a plasticiser composition containing one or more terephthalic acid dialkyl esters according to any of Claims 1 to 4 per 100 parts of PVC.

20. Extruded articles obtained from a plasticised PVC composition containing from 20 to 100 parts of one or more terephthalic acid di-alkyl esters according to any of Claims 1 to 4 per 100 parts of polyvinyl chloride.

21. A process for the manufacture of terephthalic acid di-alkyl esters by esterification of terephthalic acid with an alcohol mixture containing linear and branched C4 to C13 alcohols in which from 10 to 50 mole % (based on the alcohol mixture) are linear C4 to C 13 alcohols and the branched alcohols contain methyl branches and are free of ethyl branches and the branched alcohols contain an average of more than seven carbon atoms.
